# EUROPEAN PATENT APPLICATION

(11) **EP 0 595 659 A2**
(43) Date of publication of application: **04.05.1994**
(21) Application number: 93308688.6
(22) Date of filing: 29.10.1993
(51) Int. Cl.: C07K 15/00, C12N 15/62, A61K 37/02

(54) **Extracellular matrix receptor ligands that modulate leukocyte function**

(30) Priority: 30.10.1992 US 969700
(71) Applicant: Bristol-Myers Squibb Company, New York, N.Y. 10154 (US)
(72) Inventor: Ruffo, Alejandro A., Edmonds, WA 98020 (US); Chalupny, Jan N., Seattle, WA 98103 (US); Marken, John, Seattle, WA 98146 (US); Ledbetter, Jeffrey A., Seattle, WA 98177 (US)
(74) Representative: Jones, Alan John

(57) **Abstract**

Soluble fusion molecule containing the extracellular binding region of leukocyte receptors are described which are capable of binding to extracellular matrix associated proteins. The described lymphocyte to ligand proteins include soluble fusion molecules of 4-1BB, CD27, CD30, FAS, TNF receptor and CD40. A method of modulating leukocyte function is described in which a soluble lymphocyte between leukocyte receptors and the extracellular matrix associated protein of the target tissue.

## Description

### BACKGROUND OF THE INVENTION

The amino acid sequences of leukocyte surface molecules often contain motifs that have about 20 - 30% amino acid identity to motifs either within the molecule or in other proteins. These motifs are predicted to be evolutionarily related, have similar folding patterns and mediate similar types of interaction. A number of superfamilies have been described, containing conserved sequence motifs, such as the Ig-like domains of the immunoglobulin superfamily, fibronectin type III domains and a conserved motif found in many of the receptors for cytokines. The term domain is used where there are structural data to indicate that the motif may form a discrete structure, whereas the term motif is used where only sequence data are available.

The members of a new superfamily, the nerve growth factor receptor (NGFR) superfamily include NGFR, found on neural cells; the B-cell antigen CD40; the MRC OX-40 antigen, which is a marker of activated T cells of the CD4 phenotype; two receptors for tumour necrosis factor (TNF), called TNFR-I and TNFR-II, which are found on a variety of cell types; a cDNA (4-1BB) obtained from T-cell clones; and SFV-T2, an open reading frame in Shope fibroma virus; FAS, a polypeptide expressed in various human cells including myeloid cells, T lymphoblastoid cells and diploid fibroblasts; CD27, a T cell activation antigen; and CD30, an antigen on the surface of T and B cells which is a characteristic marker for Hodgkin's disease. The members of the family are acids, in the extracellular part of the molecule. They are not associated with any other motif type in the extracellular region, although there is a hinge-like region, characterized by a lack of Cys residues and a high proportion of Ser, Thr and Pro, in MRC OX-40, NGFR and TNFR-II. This segment is likely to be glycosylated with O-linked sugars and could have an extended structure. Alignment of all the motifs shows that they contain either four or six cysteine residues in a characteristic and conserved pattern. There is considerable variation in the rest of the sequence, although some other residues are conserved in most of the motifs. The motifs in the 4-1BB protein are less homologous but two motifs separated by truncated motif can be found. The motifs contain a variety of residues found in the nonconserved positions. Although there are some differences in the number of residues between the Cys residues, the motifs are remarkably similar in length. The cysteine-rich motifs in the NGFR superfamily are clearly distinct from cysteine-rich repeats found in other proteins, such as the low-density lipoprotein (LDL) repeat. A statistical evaluation of the sequence similarities in the NGFR family and their comparison with other cysteine-rich repeats, such as the low-density lipoprotein (LDL) repeat, is discussed in Mallett, S. et al. (1990) EMBO. J. 9:1063-1068.

A FAS polypeptide has been produced which is encoded by the cDNA for the human cell surface antigen FAS, and it has been shown that this polypeptide can mediate apoptosis (Itoh, N. et al. (1991) Cell 66:233-243). Defects in the FAS antigen have been associated with lymphoproliferative disorders in mice, producing lupus-like symptoms (Watanabe-Fukunaga, R. et al. (1992) Nature 356:314-317). Binding of TNF to the cell surface FAS antigen has been shown to mediate cytolytic activity, suggesting that the FAS antigen serves as a receptor for TNF's cytolytic effects (Yonehara, S. et al. in Tumor Necrosis Factor: Structure-Function Relationships and Clinical Applications., Basel (1992) pp. 58-65).

CD30 is a marker for Hodgkin's disease and is interchangeably referred to as the Ki-1 antigen. CD30 has 5 cysteine-rich repeat regions that are homologous with the NGFR superfamily. Although the function of CD30 in Hodgkin's disease is unknown, it is a marker of T cell and B cell activation (Durkop, H. et al. (1992) Cell 68:421-427).

The T cell activation antigen CD27 is a member of the NGFR gene family, and it may be involved in T cell proliferation and survival following activation (Camerini, D. et al. (1991) J. Immunol. 147:31653169). CD27 is a dimeric membrane glycoprotein and is found on the surface of most human T lymphocytes. Structural similarities of CD27 suggest that it belongs to a lymphocyte-specific subgroup of the NGFR family together with CD40, MRC OX40 and 4-1BB.

Functions of four members of the NGFR superfamily are known: the NGFR, FAS, and the two TNFRs are growth factor receptors. Binding of polypeptide cytokines to these receptors results in a variety of functional effects. The binding of NGF to its high-affinity receptor (which contain two polypeptide chains) significantly affects neuronal differentiation and survival (Barde, Y-A. (1989) Neuron 2:1525-1534). Both TNF-α and TNF-β bind to the two TNFRs, leading to a diverse range of effects including inflammation and tumor cell death (Beutler, B. et al. (1989) Annu. Rev. Immunol. 7:625-655; Sprang, S.R. (1990) Trend Biochem. Sci. 15:366-368). There are limited in vitro functional data on the OX-40 and CD40 antigens. Antibodies against the OX40 antigen augment the T-cell response in a mixed lymphocyte reaction (Paterson, D.J. et al. (1987) Mol. Immunol. 24:1281-1290) and antibodies against CD40 enhance B-cell proliferation in the presence of a co-activator, such as tetradecanoylphorbol acetate (TPA) or CD20 antibodies (Ledbetter, J.A. et al. (1987) J. Immunol. 138:788-794), and synergize with IL-4 in vitro to induce B-cell differentiation and IgE production (Jabara, H.H. et al. (1990) J. Exp. Med. 172:1861-1864). The 4-1BB has been characterized only as a cDNA clone found in activated T-cell clones and there are not data on the product although it appears to code for a cell surface protein (Kwon, B.S., (1989) Proc. Natl. Acad. Sci. USA 86:1963-1967). The vaccinia and Shope viral sequences are characterized only as sequences and it is not known whether the proteins are expressed (Upton, C. et al. (1987) Virology 160:20-30; Howard, S.T. et al. (1991) Virology 180:633-647).

The available evidence suggests that the NGFR superfamily cell surface proteins may function as receptors for cytokines. Those characterized so far are unusual in that they can bind to more than one ligand, and these ligands are polymeric in nature. The size of the extracellular domains of the NGFR superfamily members is relatively homogeneous compared with other superfamilies. The characterized members share 3 - 4 motifs, and in some cases a hinge-like region. Presumably, new members have yet to be defined and sequence analysis is required to see whether these features are common to all members of this superfamily.

This superfamily is unusual in that three members have been shown to bind to more than one ligand and these ligands are dimeric or trimeric in contrast to many other cytokines. Both TNFR-I and TNFR-II bind TNF-α and TNF-β with high affinity (Schall, T.J. et al. (1990) Cell 61:361-370; Smith C.A. et al. (1990) Science 248:1010-1023; Sprang, S.R. (1990) Trends Biochem. Sci. 15:366-368). These two cytokines are only 30% identical in amino acid sequence. TNF-β is thought to have similar structure to TNF-α, which contains a β sheet structure in a trimeric arrangement (Jones, E.Y. et al. (1989) Nature 338:225-228; Eck, M.J. et al. (1989) J. Biol. Chem. 264:17595-17605). Surprisingly, the two TNFRs are only 27% identical in the extracellular domains, which is the same order as that for other, functionally unrelated, members of this superfamily.

Remarkably, the two receptor molecules are homologous only within a 150 residue cysteine-rich segment presumed to be the ligand-binding domain. An analog of this sequence is found also in the receptor for nerve growth factor (NGFR), a hormone which is unlikely to have any structural resemblance to TNF. Thus, the surface of the binding scaffold presented by this cysteine-rich fold is potentially complementary to a variety of ligands.

Tumor necrosis factor is the collective name for two related cytokine hormones, TNF-α and TNF-β. TNF-α is named for its ability to induce hemorrhagic necrosis of certain murine tumors and its cytotoxicity to some tumor cell lines (Beutler, B. et al. (1988) Annu. Rev. Biochem. 57:505-518). Known also as cachectin, TNF-α is a mediator of inflammation, sepsis-induced shock and the wasting syndrome (cachexia) associated with chronic infection and neoplastic disease. A multipotent cytokine, TNF-α acts as an immunoregulator, represses lipogenic pathways in adipocytes, stimulates collagenase production in epithelial cells and is a growth factor for fibroblasts. TNF-β, or lymphotoxin, is a lymphocyte-derived homologue of TNF-α and appears to effect many of the same functions.

Consistent with its wide-ranging activities, receptors for TNF are expressed by many somatic cell types. Receptors bind both TNF-α and TNF-β (Stauber, G.B. et al. (1989) J. Biol. Chem. 264:3573-3576), but no other cytokines or growth factors, with dissociation constants in the nanomolar range. Recently it has become evident that there are at least two molecular species of TNF receptor (TNFR), a high affinity (Kd = 0.07 nM) 75-80 kDa myeloid cell type receptor and a 55-60 kDa receptor of epithelial origin with an affinity constant (Kd) of 0.3 nM (Brockhaus, M. et al. (1990) Proc. Natl. Acad. Sci. USA 87:3127-3131). The two molecules differ in their level of glycosylation, yield different peptide fragments upon tryptic digestion, and are immunologically distinct.

### SUMMARY OF THE INVENTION

The present invention is directed to soluble lymphocyte ligand proteins and methods of modulating leukocyte function by inhibiting the interaction between extracellular matrix associated proteins and lymphocytes. In this method the soluble ligand proteins are administered to a patient and bind to these extracellular matrix associated proteins. This blocks the ability of the lymphocytes to bind to the extracellular matrix receptors.

The soluble lymphocyte ligand proteins of this invention contain the soluble extracellular portions of lymphocyte surface receptors, particularly lymphocyte proteins which are members of the nerve growth factor receptor superfamily. Specific proteins of the present invention include 4-1BB fusion proteins, such as 4-1BB Rg, as well as fusion proteins of CD27, CD30, FAS, etc.

The present invention demonstrates that the soluble lymphocyte ligand proteins developed are involved in the binding of extracellular matrix associated proteins. Because interactions between leukocyte receptors and their ligands results in the modulation of leukocyte function, the inhibition of this interaction by administration of the soluble proteins of the present invention effectively alters leukocyte activity.

### BRIEF DESCRIPTION OF THE DRAWINGS

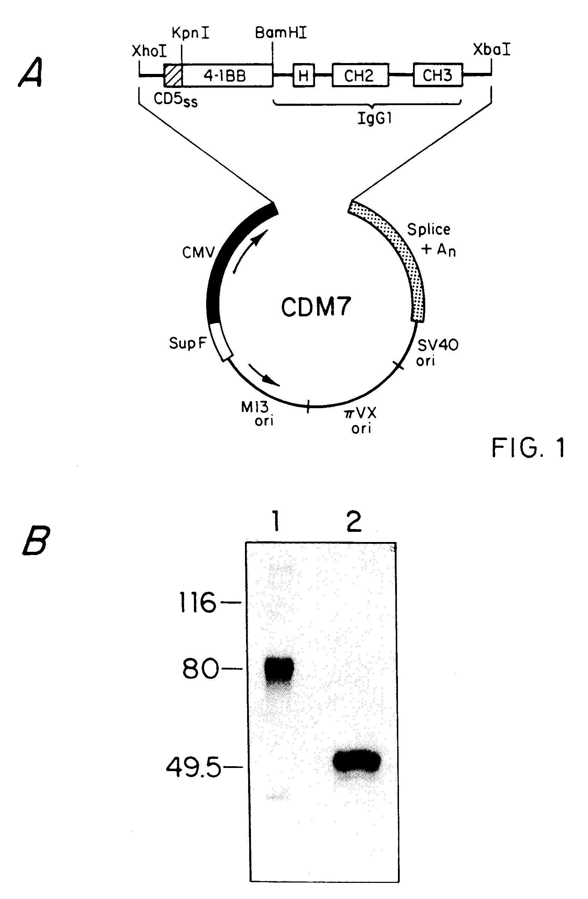

**FIGURE 1** illustrates the 4-1BB immunoglobulin fusion gene.
**A.** Elements in the mammalian expression vector CDM7 plasmid containing the 4-1BB Rg fusion gene include: a human cytomegalovirus enhancer/promoter element (CMV); and SV40 virus derived intron and polyadenylation sequences (splice + An); and SV40 virus derived origin of replication (SV40 ori); and E. coli origin of replication (π VX ori); an M13 phage derived origin of replication (M13 ori); and as a bacterial genetic marker the Sup F gene (Sup F). The 4-1BB Rg is composed of: a CDS derived amino terminal secretory signal sequence (CD5ss); a cDNA fragment encoding the extracellular domain of 4-1BB (4-1BB); and a genomic DNA fragment encoding the hinge (H) and constant domains (CH2 and CH3) of human IgG1. Unique restriction enzyme sites flanking each of the elements in the 4-1BB Rg fusion are shown.
**B.** Radiolabeled 4-1BB Rg was purified by adsorption to and elution from protein A-sepharose columns as described in materials and methods and electrophoresed under nonreducing (lane 1) or reducing (lane 2) conditions. The electrophoretic mobility of molecular mass standards are shown on the left.

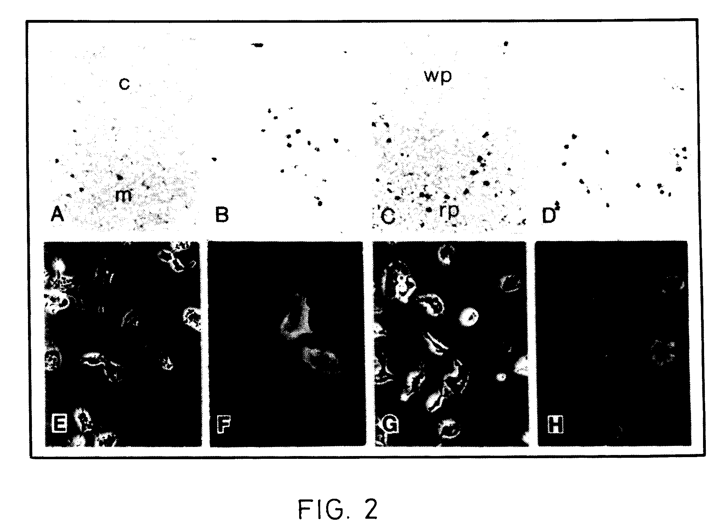

**FIGURE 2** illustrates the reactivity of 4-1BB Rg to murine tissue sections and binding of extracellular matrix proteins to COS cells expressing 4-1BB.
**A.** Immunoperoxidase localization of 4-1BB Rg binding to murine thymus in both the cortex (c) and medulla (m). Stromal cells in the medullary area of the thymus were more extensively labeled.
**B.** Reactivity of murine thymus tissue with normal human IgG1 and peroxidase-labeled goat anti-human Ig. The only reaction product observed was associated with endogenous peroxidase activity displayed by granulocytes and other cells in the thymus medulla.
**C.** Immunoperoxidase localization of 4-1BB binding to murine spleen. Both the white pulp (wp) and red pulp (rp) areas of the spleen bound the 4-1BB Rg, with more extensive binding in red pulp areas.
**D.** When normal human IgG1 was substituted for the 4-1BB Rg, deposition of reaction product was indistinguishable from endogenous peroxidase.
   Figures A - D 50X.
**E and F.** Phase and immunofluorescence micrographs depicting the binding of biotinylated laminin to COS cells transfected with 4-1BB. Arrowheads in phase micrographs identify COS cells binding laminin.
**G and H.** Phase and immunofluorescence micrographs depicting the binding of biotinylated fibronectin to COS cells transfected with 4-1BB. Arrowheads in phase micrograph identify COS cells binding fibronectin.
   Figures E - H 200 X.

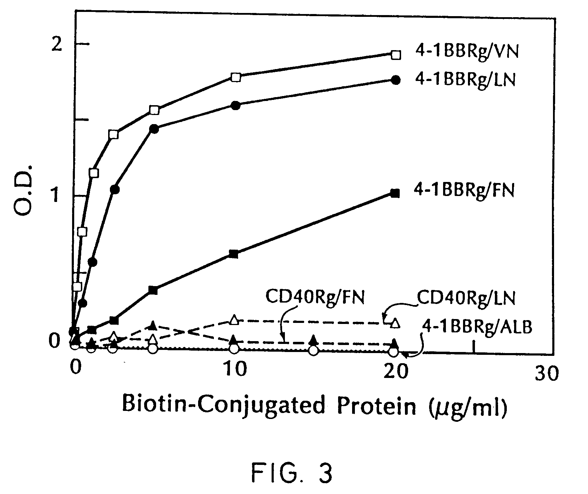

**FIGURE 3** illustrates the binding of 4-1BB Rg to extracellular matrix proteins. 4-1BB Rg was immobilized on plastic coated with affinity purified goat anti-human IgG antibodies and its ability to bind to increasing amounts of biotin-conjugated fibronectin, vitronectin, laminin or BSA was measured using an ELISA assay, as described in the materials and methods section. As a control the binding of a similarly immobilized CD40 Rg fusion protein to biotin-conjugated fibronectin and laminin was monitored. The data are expressed as the mean of triplicate samples. The SD were <1%. The data shown are representative of at least three separate experiments.

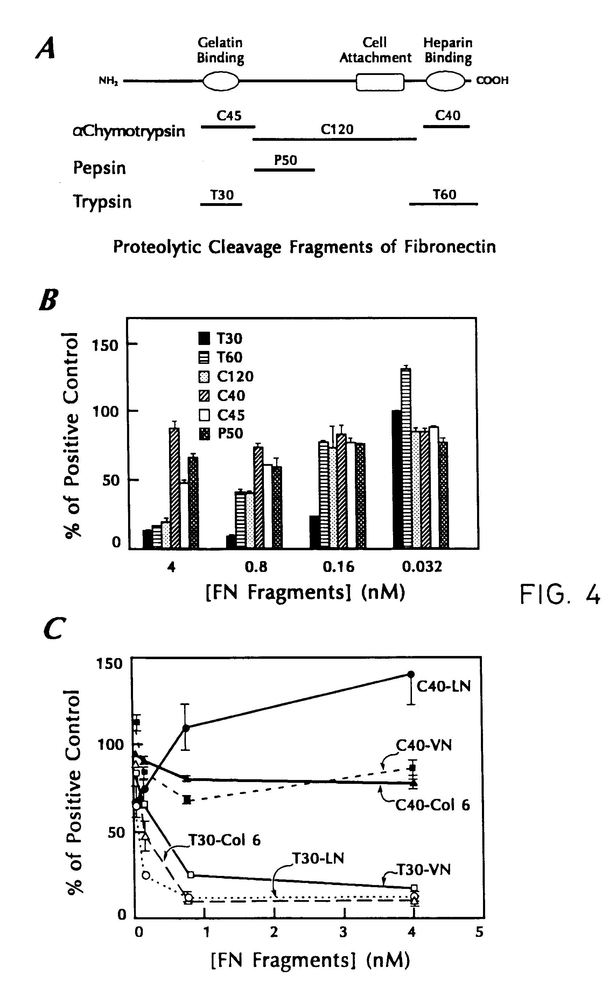

**FIGURE 4** illustrates that the binding of 4-1BB Rg to fibronectin is mediated by multiple fibronectin domains. Extracellular matrix proteins bind to 4-1BB Rg via overlapping binding sites.
**A.** Schematic representation of the human fibronectin polypeptide and the proteolytic fragments used in the blocking experiments. The two trypsin derived fragments T30 and T60 have an approximate molecular mass of 30 and 60 kD, respectively. The α-chymotrypsin derived fragments C45, C120, and C40 have an approximate molecular mass of 456, 120, and 40 kD, respectively. The pepsin derived fragment P50 has an approximate molecular mass of 50 kD. These peptides were obtained from Telios Pharmaceuticals Inc. (San Diego, CA).
**B.** The ability of the different proteolytic fragments of fibronectin to block the binding of fibronectin to 4-1BB Rg immobilized on plastic was measured using an ELISA assay as described in the material and methods section. Each fragment was tested at four different concentrations. The fibronectin fragments tested include T30, T60, C120, C40, C45 and P50. The data are expressed as the mean of triplicate samples. SD are shown. The data shown are representative of at least three separate experiments.
**C.** The ability of increasing amounts of the T30 and C40 fragments of fibronectin to inhibit the binding of laminin, vitronectin and collagen VI to 4-1BB Rg immobilized on plastic was measured by the ELISA method as described in the materials and methods section. The data are expressed as the means of triplicate samples. The data shown are representative of at least three separate experiments.

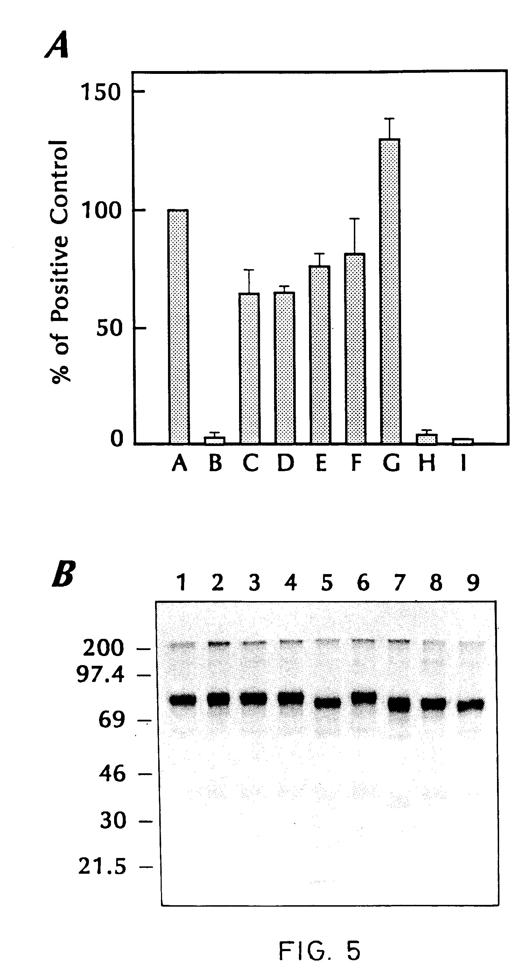

**FIGURE 5** illustrates glycosaminoglycan blocking of the binding of 4-1BB to extracellular matrix proteins. 4-1BB Rg is modified with N-linked and O-linked oligosaccharides but not glycosaminoglycans.
**A.** The ability of the anionic carbohydrate polymers fucoidan (H) and dextran sulfate (D), and the glycosaminoglycans heparin (C), chondroitin (E), and hyaluronan (F) and desulfated-heparin (G) to block the binding of fibronectin to COS cells transfectants expressing 4-1BB was tested as described in materials and methods. Binding is shown as % binding relative to the binding of 4-1BB COS cell transfectants to fibronectin in the absence of inhibitors (A). Controls included the binding of phycoerythrin conjugated strepavidin alone to COS cells expressing 4-1BB (B) and the binding of COS cell transfectants expressing CD40 to fibronectin (I). The data shown are expressed as the means of triplicate scans. The SD are calculated as described in the materials and methods. The data shown are representative of at least three separate experiments.
**B.** Radiolabeled purified 4-1BB Rg was treated with chondroitin ABC lyase, heparinase, heparatinase, keratanase, hyaluronidase, N-glycanase, O-glycanase, and neuraminidase (lanes 2-9) as described in materials and methods section. The electrophoretic mobility of the treated 4-1BB Rg protein was compared to that of untreated 4-1BB Rg protein (lane 1) using SDS-PAGE as described in materials and methods section. The electrophoretic mobility of molecular mass standards are shown on the left.

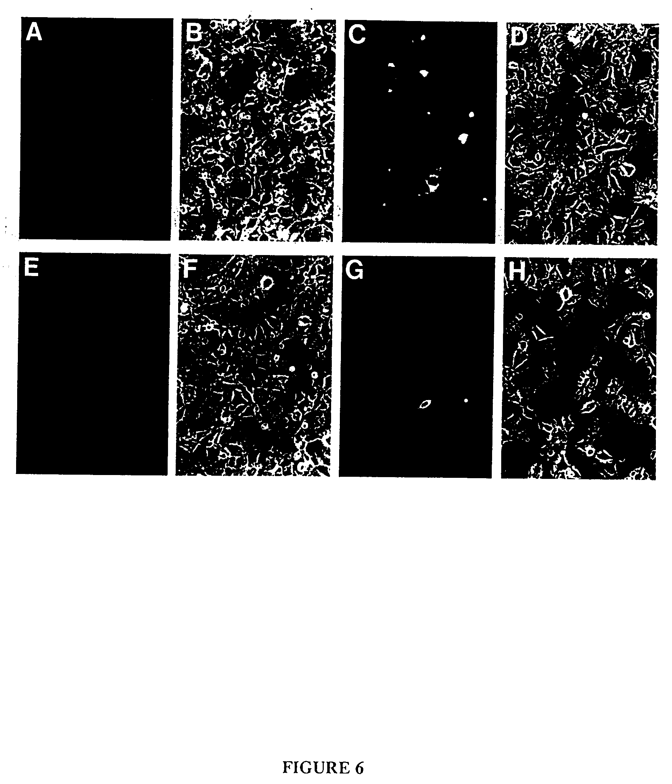

**FIGURE 6** illustrates mock transfection of COS cells stained with either anti-CD30 mAb (A, B) or anti-CD27 mAb (E, F) viewed under fluorescence (A, E) or phase contrast microscopy (B, F). COS cells transfected with CD30Rg (C, D) or CD27Rg (G, H) are similarly stained and viewed under fluorescence (C, G) or phase contrast microscopy (D, H), respectively.

### DESCRIPTION OF PREFERRED EMBODIMENTS

The present invention is directed to soluble fusion molecules of T cell surface ligands that are capable of inhibiting the interaction of leukocytes with proteins associated with the extracellular matrix of target tissue, and to methods for the inhibiting of leukocyte action.

Soluble fusion molecules of the present invention include the extracellular domain of a T cell surface ligand fused to an immunoglobulin chain (Rg). Particularly preferred fusion molecules of the present invention include 4-1BBRg, CD30Rg, CD27Rg, CD40Rg and TNFRg.

In order to more clearly describe the present invention and its embodiments the following definitions are included.

As used herein, the term "soluble lymphocyte ligand protein" and grammatical variations thereof refers to a fusion protein composed of an extracellular region of a lymphocyte receptor protein such as 4-1BB, CD30, CD27, FAS, MRC OX40, TNF receptor (TNFR), etc. fused to a soluble structural protein such as in immunoglobulin (Rg) or T cell receptor region. Illustrative soluble lymphocyte ligand proteins of the present invention include 4-1BBRg, CD27Rg, and FAS Rg and the like.

As used herein, the term "extracellular matrix associated proteins" refers to proteins found within the extracellular matrix of tissues and include fibronectin, laminin, collagen type VI, and TNF-like molecules associated therewith.

"Cloning vector", as used herein, is any plasmid or virus into which a foreign DNA may be inserted to be cloned.

"Plasmid", as used herein, is an autonomous self-replicating extra-chromosomal circular DNA.

"Open Reading Frame" (ORF), as used herein, is a DNA sequence which is (potentially) translatable into protein.

"Gene (cistron)", as used herein, is the segment of DNA that encodes the sequence of a peptide chain; it can include regions preceding and following the coding region (leader and trailer) as well as intervening sequences (introns) between individual coding segments (exons).

"Expression", as used herein, is the process undergone by a structural gene to produce a peptide or protein. It is a combination of transcription and translation.

As used herein, the term "base pair" (bp) is a partnership of adenine (A) with thymine (T), or of cytosine (C) with guanine (G) in a DNA double helix.

As used herein, the term "expression vector" is any plasmid or virus into which a foreign DNA may be inserted and/or expressed.

As used herein, the term "downstream" identifies sequences proceeding further in the direction of expression; for example, the peptide coding region of a gene is downstream from the initiation codon or in the 3' direction away from the gene; upstream is 5' to the sequence in question.

As used herein, the term "polymerase chain reaction" (PCR) refers to the amplification of DNA molecules by the successive use of a temperature stable DNA polymerase to copy the DNA chain, separating the complementary chains by heating, adding primers and repeating the process about 30 times to produce approximately 10⁹ copies of the DNA. By use of the PCR technique, minute amounts of DNA can be amplified to produce sufficient DNA for use in various procedures.

The term "unit dose" refers to physically discrete units suitable as unitary dosages for animals, each unit contains a predetermined quantity of active material calculated to produce the desired therapeutic effect in association with the required diluent; that is, a carrier or vehicle. The specifications for the novel unit dose of this invention are dictated by, and are directly dependent upon, (a) the unique characteristics of the active material and the particular therapeutic effect to be achieved, and (b) the limitations inherent in the art of compounding such active material for therapeutic use.

The term "correspond" in its various grammatical forms, as used herein and in the claims in relation to peptide sequences means the peptide sequence described plus or minus up to 3 amino acid residues at either or both of the amino and carboxy termini and containing only conservative substitutions in particular amino acid residues along the peptide and/or polypeptide sequence.

The term "conservative substitution" as used above denotes that one amino acid residue has been replaced by another, biologically similar residue. Examples of conservative substitutions include the substitutions of one hydrophobic residue such as Ile, Val, Leu, or Met for another, or the substitution of one polar residue for another such as between Arg and Lys, between Glu and Asp or between Gln and Asn, and the like.

In some instances the replacement of an ionic residue by an oppositely charged ionic residue such as Asp by Lys has been determined conservative in the art in that those ionic groups are thought to merely provide solubility assistance. In general, however, since the replacements discussed herein are on a relatively short region, replacement of an ionic residue by another ionic residue of opposite charge is considered herein to be a "radical replacement" as are replacements by nonionic and ionic residues, and bulky residues such as Phe, Tyr or Trp and less bulky residues such as Gly, Ile and Val.

The terms "nonionic" and "ionic" residues are used herein in their usual sense to designate those amino acid residues that either bear no charge or normally bear a charge, respectively, at physiological pH value. Exemplary nonionic residues include Thr and Gln, while exemplary ionic residues include Arg and Asp.

The phrases "pharmaceutically acceptable salts" and "physiologically tolerable salts", as used interchangeably herein, refer to non-toxic alkali metal, alkaline earth metal and ammonium salts used in the pharmaceutical industry, including the sodium, potassium, lithium, calcium, magnesium and ammonium salts and the like that are prepared by methods well-known in the art. The phrase also includes non-toxic acid addition salts that are generally prepared by reacting the compounds in this invention with a suitable organic or inorganic acid. Representative salts include the hydrochloride, hydrobromide, sulfate, bisulfate, acetate, oxalate, valerate, oleate, laureate, vorate, benzoate, lactate, phosphate, tosylate, citrate, maleate, fumarate, succinate, tartrate and the like.

As used herein, the term "cellular adhesion molecule" refers to specific inflammatory cell surface molecules that are recognized and bind to vascular endothelium and/or granulocytes.

As used herein, the term "operatively attached" refers to the linkage of groups in a manner such that the binding affinity of the group is not inhibited by the attachment.

As used herein, the term "IgG constant region" refers to domains of the gamma chain of the IgG molecule that are adjacent to the variable region that corresponds to the first 107 amino acids of the gamma chain or fragments thereof. The four domains within the gamma chain constant region are designated CH₁, H, CH₂, and CH₃. CH₁ is adjacent to the variable region and encompasses amino acid residues 114 through 223. H (hinge; residues 224-245) is adjacent to CH₁ and contains the cysteine residues that form the disulfide bonds which covalently link the two immunoglobulin heavy chains. CH₂ is adjacent to the hinge and encompasses amino acid residues 246 through 361, followed by CH₃ which contains amino acid residues 362 through 496.

As used herein, the term "library" refers to a large random collection of cloned DNA fragments obtained from the transcription system of interest. The gene library was then screened with labeled cDNA probes.

A carrier is a material useful for administering the active compound and must be "acceptable" in the sense of being compatible with the other ingredients of the composition and not deleterious to the recipient thereof.

The pharmaceutical compositions are prepared by any of the methods well known in the art of pharmacy all of which involve bringing into association the active compound and the carrier therefor.

For therapeutic use, the agent utilized in the present invention can be administered in the form of conventional pharmaceutical compositions. Such compositions can be formulated so as to be suitable for oral or parenteral administration, or as suppositories. In these compositions, the agent is typically dissolved or dispersed in a physiologically tolerable carrier.

As an example, the compounds of the present invention can be utilized in liquid compositions such as sterile suspensions or solutions, or as isotonic preparations containing suitable preservatives. Particularly well suited for the present purposes are injectable media constituted by aqueous injectable isotonic and sterile saline or glucose solutions. Additional liquid forms in which the present compounds may be incorporated for administration include flavored emulsions with edible oils such as cottonseed oil, sesame oil, coconut oil, peanut oil, and the like, as well as elixirs and similar pharmaceutical vehicles.

The present compounds can also be used in compositions such as tablets or pills, preferably containing a unit dose of the compound. To this end, the agent (active ingredient) is mixed with conventional tabletting ingredients such as corn starch, lactose, sucrose, sorbitol, talc, stearic acid, magnesium stearate, dicalcium phosphate, gums or similar materials as non-toxic, physiologically tolerable carriers. The tablets or pills of the present compositions can be laminated or otherwise compounded to provide unit dosage forms affording prolonged or delayed action.

It should be understood that in addition to the aforementioned carrier ingredients the pharmaceutical formulation described herein can include, as appropriate, one or more additional carrier ingredients such as diluents, buffers, flavoring agents, binders, surface active agents, thickeners, lubricants, preservatives (including antioxidants) and the like, and substances included for the purpose of rendering the formulation isotonic with the blood of the intended recipient.

The tablets or pills can also be provided with an enteric layer in the form of an envelope that serves to resist disintegration in the stomach and permits the active ingredient to pass intact into the duodenum or to be delayed in release. A variety of materials can be used for such enteric layers or coatings, including polymeric acids or mixtures of such acids with such materials as shellac, shellac and acetyl alcohol, cellulose acetate, and the like. A particularly suitable enteric coating comprises a styrene-maleic acid copolymer together with known materials that contribute to the enteric properties of the coating.

As used herein, the terms "label", "indicating means" and "labeled indicating means", in their various grammatical forms refer to single atoms and molecules that are either directly or indirectly involved in the production of a detectable signal to indicate or detect the presence of a reaction product. Such labels are themselves well known in clinical diagnostic chemistry and constitute a part of this invention only insofar as they are utilized with otherwise novel methods and/or systems.

The indicating means can be a fluorescent labeling agent that chemically binds to antibodies or protein antigens without denaturing them to form a fluorochrome (dye) that is a useful immunofluorescent tracer. Suitable fluorescent labeling agents are fluorochrome, such as fluorescein isocyanate (FIC), fluorescein isothiocyanate (FITC), 5-dimethylamine-1-naphthalene sulfonyl chloride (DANSC), tetramethylrhodamine isocyanate (TRITC), lissamine and the like. Immunofluorescence analysis techniques are well known in the art, and for example, is described in DeLuca, "Immunofluorescence Analysis" in Immunofluorescence Analysis, Marchalonis et al., (1982) eds., John Wiley & Sons, Ltd., pp. 189-231, which is incorporated herein by reference.

Other preferred indicating means are colorimetric agents and enzymes, such as horseradish peroxidase, glucose oxidase or the like, linked as described above, as well as radioactive elements, preferably an element that produces gamma ray emissions. Elements which emit gamma rays, such as ¹²⁴I, ¹²⁵I, ¹²⁸I, ¹³²I, and ⁵¹Cr represent one class of radioactive indicating groups. Another group of useful labeling means are those elements such as ¹¹C, ¹⁸F, ¹⁵O and ¹³N which emit positrons. The positrons so emitted produce gamma rays upon interaction with electrons present.

The T cell activation marker 4-1BB was isolated by differential screening of a cDNA library prepared from the lymphokine activated cytotoxic T cell line L3 (Kwon, B.S. et al. (1987) Proc. Natl. Acad. Sci. USA 84:2896-2900). Nucleotide sequencing of three overlapping cDNA cell lines of the 4-1BB gene revealed that it encodes a type I membrane protein of 256 amino acids. The extracellular domain of 4-1BB can be divided into two regions; a cysteine-rich region and a serine/threonine rich region. Published comparison between the predicted 4-1BB amino acid sequence and those of other known proteins show that the extracellular domain of 4-1BB has limited homology to members of the nerve growth factor receptor family (NGFR) (Mallett, S. et al. (1991) Immunol. Today 12:220-223) and that its cytoplasmic domain contains a putative binding site for the lymphocyte specific protein tyrosine kinase p561ck (Shaw, A.S. et al. (1990) Mol. Cell. Biol. 10:1853-1862). RNA blot studies showed that mRNAs encoding 4-1BB are expressed by a number of activated T-cell lines (Kwon, B.S. et al. (1987) Proc. Natl. Acad. Sci. USA 84:2986-2900; Kwon, B.S. et al. (1989) Cell. Immunol. 121:414-422). As a first step in elucidating the function of 4-1BB the 4-1BB ligand(s) were identified and characterized.

Immunohistochemical studies with 4-1BB recombinant immunoglobulin fusion protein (4-1BB Rg) revealed that 4-1BB Rg bound extensively to a variety of tissues and suggested that extracellular matrix components might be involved. The interaction between 4-1BB and several extracellular-matrix proteins including fibronectin, laminin, vitronectin, and collagen VI was confirmed using COS cell transfectants expressing 4-1BB. Ligand blocking experiments showed that various anionic carbohydrate polymers such as fucoidan, dextran sulfate or the glycosaminoglycans heparin, chondroitin sulfate, and hyaluronan could block the extracellular matrix protein-4-1BB interaction and suggests that carbohydrates participate in this interaction.

Immunohistochemical studies showing that 4-1BB Rg bound to virtually all tissues examined suggested that extracellular matrix components might be serving as ligands for this molecule. The interaction between extracellular matrix proteins and 4-1BB was confirmed using COS cell transfectants. COS cells expressing 4-1BB were found to bind fibronectin, laminin, vitronectin and collagen VI but not collagen I while those expressing CD40 (Stamenkovic, I. et al. (1989) EMBO J. 8:1403-1410) bound none of these proteins. This binding was found to be concentration dependent and saturable. Fibronectin binding to 4-1BB was not mediated by the RGD or CS-1 sequences which have been shown to mediate fibronectin binding to lymphocyte integrins (Springer T.A. (1990) Nature 346:425-434). Binding studies with fibronectin peptides showed that distinct regions of the fibronectin molecule can bind to 4-1BB. Four out of the six proteolytic cleavage fragments of fibronectin were able to inhibit the 4-1BB-fibronectin interaction in a concentration dependent fashion. However, of these, only one, C120, contains sequences known previously to mediate fibronectin-cell attachment (Pytela, R. (1985) Cell 40:191-198).

Blocking studies with the T30 and T60 proteolytic fragments of fibronectin suggest that the binding sites of 4-1BB for all the extracellular matrix proteins are overlapping. T30 and T60 completely blocked the binding of laminin, vitronectin, and collagen VI to 4-1BB.

Extracellular matrix proteins are known to bind to proteoglycans by an Arg-Gly-Asp (RGD) and CS-1 independent mechanism (Ruoslahti, E. et al. (1991) Cell 64:867-869; Ruoslahti, E. (1988) Ann. Rev. Cell. Biol. 4:229-255). Using blocking experiments, the possibility was investigated that the 4-1BB-extracellular matrix protein interaction could be inhibited with glycosaminoglycans and other anionic carbohydrate polymers and that 4-1BB Rg itself is a proteoglycan. The interaction between 4-1BB and extracellular matrix proteins was completely blocked by the anionic carbohydrate polymer fucoidan, and partially blocked by the glycosaminoglycans, heparin sulfate, chondroitin sulfate and hyaluronan, and by the oligosaccharide dextran sulfate. Conversely, de-sulfated-heparin and the simple sugars, fucose, mannose, glucose, glucose-6-sulfate, N-acetyl-glycosamine, N-acetyl-galactosamine, and N-acetyl-neuraminic acid did not affect the interaction. These results suggest that carbohydrates might be involved in mediating the interaction between 4-1BB and extracellular matrix proteins.

Digestion of 4-1BB Rg with chondroitin ABC lyase, hyaluronidase, heparinase, and heparatinase gave no evidence for the presence of these glycosaminoglycans on 4-1BB Rg. Keratanase digestion reduced the molecular mass of 4-1BB Rg, and 4-1BB Rg reacted with anti-keratin mAb suggesting that 4-1BB Rg contains carbohydrate moieties with the sequence GlcNac (β-1,3)Gal(β-1,4)GlcNAc (Fukuda, M.N. (1981) J. Biol. Chem. 256:3900-3905) and possibly keratan sulfate. Digestion with N-glycanase, O-glycanase and neuraminidase show that 4-1BB contains N-linked carbohydrates, O-linked carbohydrates and sialic acid. These results indicate that 4-1BB is a glycoprotein.

The interaction between 4-1BB and extracellular matrix proteins was unexpected, given the previous report that 4-1BB is homologous to the members of the nerve growth factor receptor (NGFR) family (Mallett, S. et al.(1991) Immunol. Today 12:220-223; Smith, C.A. et al. (1990) Science 248:1019-1023). This family of proteins consists of the NGFR (Radeke, M.J. et al. (1987) Nature 325:593-597) two receptors for tumor necrosis factor (Loetscher, H. et al. 1990) Cell 61:351-359; Schall, T.J. et al. (1990) Cell 61:361-370; Smith C.A. et al. (1990) Science 248:1019-1023), the B-cell antigens CD30 (Durkop, H. et al. (1992) Cell 68:421-427), CD40 (Stamenkovic, I. et al. (1989) EMBO J. 8:1403-1410) and FAS (Itoh, N. et al. (1991) Cell 66:233-243), the T cell surface antigens OX-40 (Mallett, S. et al. (1990) EMBO J. 9:1063-1068) and CD27 (Camerini, D. et al. (1991) J. Immunol. 147:3165-3169) and the open reading frame of the Shope fibroma virus designated SFV-T2 (Sprang, S.R. (1990) Trends in Biochem. Sci. 15:366 368). Ligands for only four of these proteins (NGFR, TNFR-1 and II and CD40) have been identified (Sprang, S.R. (1990) Trends in Biochem. Sci. 15:366-368; Bothwell, M. (1991) Cell 65:915-918) and none have previously been reported to bind to extracellular matrix proteins. Furthermore, the CD40 Rg and COS cell transfectants expressing CD40 (Stamenkovic, I. et al. (1989) EMBO J. 8:1403-1410) used as controls showed no binding to extracellular matrix proteins.

These observations led to a reexamination of the homology between 4-1BB and other proteins in the nBRF and SWISPROT data bases using the FASTP program. In addition to the previously reported homology, 4-1BB is significantly homologous to the Cys rich domains of the short arms of laminin (Sasaki, M. et al. (1988) J. Biol. Chem. 263:16536-16544), the proteoglycan perlecan (Noonan, D.M. et al. (1991) J. Biol. Chem. 266:22939-22947) and the human β5 integrin chain (Cheresh, D.A. et al. (1989) Cell 57:59-69; McLean, J.W. et al. (1990) J. Biol. Chem. 265:17126-17131). Sequence alignment shows that the extracellular domain of 4-1BB can be divided into two laminin- like Cys rich domains each containing 7 Cys residues. These Cys residues are conserved among 4-1BB and the Cys rich domains of laminin and perlecan (data not shown). Interestingly, a non-RGD sequence responsible for mediating cell attachment has been mapped to one such Cys rich domain on the laminin B1 chain (Graf, J. et al. (1987) Cell 48:989-996). The possible significance of the homology between 4-1BB and the human β5 integrin chain is less clear. The homology algorithm matches a number of residues in the two sequences, however most of them are not conserved between members of the integrin family of proteins. In addition, although β5 integrins are known to bind to vitronectin and fibronectin, this adhesive interaction is mediated by RGD sequences (Springer, T.A. (1990) Nature 346:425-434). These observations in conjunction with the binding studies, suggest that 4-1BB might be distantly related to more than one gene family.

The results presented here indicate that 4-1BB is a T cell surface protein involved in the binding of extracellular matrix proteins. An increasing body of evidence indicates that the interaction between leukocyte extracellular matrix receptors and their ligands result in the modulation of leukocyte function (Springer, T.A. (1990) Nature 346:425-434; Nojim Y. et al. (1990) J. Exp. Med. 172:11851192). This work suggests that the 4-1BB-extracellular matrix interaction results in the modulation of T cell function and that interactions between the cytoplasmic domain of 4-1BB and p56^{lck} are involved in this process. Soluble 4-1BB and antibodies to 4-1BB could be used to identify conditions under which normal T cells express high levels of 4-1BB and/or to identify cell lines which express high levels of this protein. Once these cells have been identified, the role of the interaction between extracellular matrix proteins and 4-1BB can be further characterized.

In order to further clarify and enable the present invention as illustrated in the Examples described herein below, a general description of the materials and methods utilized in producing the results disclosed is presented. These materials and methods illustrate the technology utilized and are not intended to be limiting of the present invention. Other variations and modifications of these methods are well-known and are contemplated by this invention.

In addition to CD40 and 4-1BB, soluble immunoglobulin fusion proteins of other members of the TNF gene family expressed by leukocytes have been prepared and are contemplated by the present invention. Figure 6 illustrates COS cell transfectants expressing fusion proteins of the T cell antigen CD27, and the B cell antigen CD30. A similar soluble immunoglobulin fusion molecule has been prepared for the leukoycte antigen FAS. These proteins were prepared in a maner analogous to that utilized for preparation of CD40 and 4-1BB fusion proteins. These fusion proteins can be utilized to inhibit leukocyte interactions thereby providing a method for modulating immune responses in a clinical setting. Similar to studies with CD40 and 4-1BB, these proteins have ligands which are related to TNF as has been shown for CD40 and/or bind to extracellular matrix associated proteins as does 4-1BB. The fusion proteins of the present invention will be utilized to isolate specific ligands which may modulate immune responses.

### Preparation Of Full Length 4-1BB cDNA

The full length 4-1BB cDNA sequence was amplified using the polymerase chain reaction (PCR) method using the conditions and reagents suggested by the vendor (Cetus). A 4-1BB synthetic primer encoding sequences immediately upstream of the signal sequence and including a Hind III site with the following sequence was synthesized:

5'-GCG ACG CGT AAG CTT GTC CTG TGC ATG TGA CAT TTC-3'
(Sequence I.D. No. 1).

A reverse primer containing a Xho I restriction site with the following sequence was synthesized:

5'-CAC TCG AGC GGC CGC TCA CAG CTC ATA GCC TCC TCC-3'
(Sequence I.D. No. 2).

Full length 4-1BB PCR products were digested with Hind III and Xho I and ligated to Hind III-Xho I-cut CDM8 vector (Seed, B. (1987) Nature 329:840-842).

### Preparation Of 4-1BB-Rg

The soluble 4-1BB-immunoglobulin fusion protein (4-1BB-Rg) used in this study was created by isolating the cDNA fragment encoding the extracellular domain of 4-1BB by PCR as described (Aruffo, A. et al. (1990) Cell 61:1303-1313). A 4-1BB primer encoding the sequence immediately downstream of the signal peptide cleavage point and including a Kpn I site was synthesized with the following sequence:

5'-CCG CGG GTA CCC GTG CAG AAC TCC TGT GAT AAC TGT-3'
(Sequence I.D. No. 3).

A reverse primer containing a Bam HI restriction site was synthesized with the following sequence:

5'-CCG CTA CGT AGG ATC CTG CAA GGA GTC CCC TCC TGG-3'
(Sequence I.D. No. 4).

The 4-1BB extracellular region PCR product was digested with Bam H1 and Kpn I and ligated to KpnI-Bam H1-cut CD5-IgG1 vector (Aruffo, A. et al. (1990) Cell 61:1303-1313). The CD40 cDNA and the CD40-immunoglobulin fusion protein (CD40 Rg) used in these studies as controls have been previously described (Stamenkovic, I. et al. (1989) EMBO J 8:1403-1410). The resulting constructs were transfected into COS cells and the desired fusion protein was recovered from the supernatant as described (Aruffo, A. et al. (1990) Cell 61:1303-1313).

### 4-1BB Immunohistology Studies

The techniques used for light microscopic immunohistochemistry have been described in detail (Farr, A.G. et al. (1981) Immunol. Methods 47:129). Freshly cut 6 µm cryostat sections of murine thymus, lymph node, spleen or skin were mounted on amino alkylsilane-treated slides (Rentrop, M. et al. (1986) Histochem. J. 18:271) and allowed to air dry for at least two hours before fixation (Guan, J.L. et al. (1990) Cell 60:53-61; Nojim, Y. et al. (1990) J. Exp. Med. 172:1185-1192; Ruoslahti, E. (1988) Ann. Rev. Biochem. 57:375-413; Ruoslahti, E. et al. (1991) Cell 64:867-869; Ruoslahti, E. (1988) Ann. Rev. Cell. Biol. 4:229-255; Doege, K. (1986) J. Biol. Chem. 262:17757-17767) minutes in acetone at -20° C. After rinsing in PBS, the sections were incubated for 1 hour at room temperature with 4-1BB Rg or human IgG1 diluted in PBS containing Ca⁺² and Mg⁺² (2mM each). All subsequent solutions and diluents contained divalent cations. After several washes in PBS, slides were then incubated with peroxidase-labeled goat anti-human IgG1 antibodies using the same conditions as employed for the primary antibodies. Peroxidase activity was demonstrated using 3, 3'-diaminobenzidine as the electron donor. Slides were then hydrated, mounted with coverslips, and photographed without additional counterstaining.

### Binding Of Purified 4-1BB-Rg Matrix Molecules

Binding of the purified 4-1BB-Rg to murine fibronectin, rat laminin, human vitronectin, human collagen type VI and I (Telios Pharmaceuticals) and bovine serum albumin (BSA) (Sigma) was tested using an ELISA method. Polystyrene 96-well plates were coated with affinity purified goat anti-human IgG Fc portion (Cappel) diluted to 5 µg/ml in PBS, 1 mM CaCl2, 1 mM MgCl2 (PBS/Ca²⁺/Mg²⁺) for one hour at 22° C. The wells were blocked with 10X Specimen Diluent Concentrate (Genetic Systems) diluted 1:10 with ddH₂0 overnight at 4 °C. Wells were washed and treated with 100 µl per well 4-1BB or CD40-Rg diluted in (PBS/Ca²⁺/Mg²⁺) at the appropriate concentrations (1.0 µg/ml for laminin) for one hour at 22 °C. Wells were treated with biotin-conjugated extracellular matrix proteins diluted in PBS/Ca²⁺/Mg²⁺ titrated from 20 µg/ml with 1:2 dilutions to 0.06 µg/ml for one hour at 22 °C. Bound material was detected using peroxidase-conjugated avidin (Sigma) diluted 1:500 in 1X Specimen Diluent Concentrate as above for one hour at 22 °C. Wells were washed and treated with EIA Chromogen Reagent (Genetic Systems) diluted 1:100 in EIA Buffered Substrate (Genetic Systems) for 15 min. at 22 °C. The colormetric reaction was quenched by adding Stop Buffer (Genetic Systems) and the optical density was measured on an ELISA reader at dual wavelengths 450,630.

Blocking of the binding of 4-1BB-Rg to extracellular matrix proteins was tested using the ELISA method described above with the following alterations: After coating the wells with 4-1BB-Rg or CD40-Rg, fibronectin tryptic fragments were diluted to 4 nM, 0.8 nM, 0.16 nM, and 0.032 nM, and RGD and CS-1 peptides were diluted to 500 µg/ml, 100 µg/ml, 20 µg/ml and 4 µg/ml in PBS/Ca²⁺/Mg²⁺ added at 100 µl/well and incubated for one hour at 22 °C, after which biotin-conjugated extracellular matrix proteins were added as above to the well without washing and incubated for another hour at 22 °C.

### Binding of 4-1BB Expressed In COS Cells To Matrix Molecules

CDM8 vectors containing either the full length 4-1BB cDNA or the full length CD40 cDNA were transfected into COS cells using DEAE dextran method (Aruffo, A. et al. (1990) Cell 61:1303-1313). Twenty-four hours post-transfection, cells were trypsinized, seeded onto 15 x 35 mm plates and allowed to grow another twenty-four hours. Before staining, cells were washed with staining buffer (PBS containing 2% FBS, 1 mM CaCl₂, 1mM MgCl₂). Cells were incubated with 4 µg of biotin-conjugated extracellular matrix protein for one hour at 22 °C. Cells were washed twice with staining buffer and incubated with 5.0 µg of fluorescein isothiocyanate (FITC) conjugated avidin (Molecular Probes) or 1.0 µg of phycoerythrin-conjugated avidin (Biomeda) for 30 min. at 4 °C. Cells were washed twice with staining buffer and examined by fluorescence microscopy.

In blocking experiments, cells were washed with staining buffer and incubated with 400 µg of the simple or 100 µg of the complex carbohydrates or polymers for one hour at 22 °C. Four µg of biotin-conjugated extracellular matrix protein was then added without washing and incubated for one hour at 22 °C. Staining proceeded as above. Fluorescence of COS cells expressing 4-1BB or CD40 was measured with an ACAS 570 microscope (Meridian) using a single image program and a combination of two filters: a diachroic filter which collects light longer than 515 nm, and a long pass filter which collects light longer than 605 nm. All 35 mm² plates contained cells at an equal density. Each scan measured the average fluorescence of a 1.8 mm² area, and each plate was scanned three times. Background fluorescence was subtracted by removing all areas of fluorescence smaller than 400 um². Each scan was expressed as the percent of the positive control (4-1BB expressing COS cells stained with biotinylated FN plus phycoerythrin-conjugated avidin), and the scans of each sample were averaged to generate a mean and standard deviation.

### 4-1BB-Rg Digestion

COS cells transfected with either CD40-Rg or 4-1BB-Rg cDNAs were incubated in Cys/Met free DMEM for 30 min. before the addition of 1.0 mCi of ³⁵S-cysteine and 1.0 mCi ³⁵S-methionine (Amersham). After 18 hours Rg fusion proteins were purified as described (Aruffo, A. et al. (1990) Cell 61:1303-1313).

³⁵S-labeled 4-1BB-Rg or CD40-Rg were digested with heparinase (0.017 U/ml), heparatinase (ICN, 0.017 U/ml), keratinase (ICN, 0.33 U/ml) or hyaluronidase (Sigma, 17 U/ml) in PBS at pH 7.0, 7.0, 7.4 and 7.4, respectively for 18 hours at 37 °C. Chondroitin ABC lyase (ICN) digestions were performed with 1.0 U/ml of enzyme in 40 mM Tris-HCl (pH 8.6) containing 40 mM sodium acetate (NaOAc) for 18 hours at 37 °C. N-glycanase (Genzyme) digestions were performed with 35 U/ml enzyme in 0.1 Tris-HCl (pH 8.0) for 18 hours at 37 °C. Neuraminidase (Genzyme) digestions were done with 0.4 U/ml enzyme in 0.1 M NaOAc (pH 6.5) containing 10 mM Ca(OAc)₂ for 2 hours at 37 °C. O-glycanase (Genzyme) digestions (0.17 U/ml) were performed on neuraminidase treated fusion proteins for 18 hours at 37 °C. Following digestions, SDS gel sample buffer was added and the digests subjected to SDS-PAGE analysis. Gels were fixed, dried and exposed to film.

### Western Blot Analysis

Proteins were electrophoresed in a 7.5% polyacrylamide gel, then electroblotted onto nitrocellulose for 1 hour at 80 mA. The membrane was blocked with 5% nonfat milk in PBS for 18 hours before including an anti-keratan sulfate antibody (5D4, ICN) diluted 1:100 in PBS 0.05% Tween-20 (PBS-Tween™) for 2 hours at 22 °C. After one wash with the PBS-Tween™ solution the membrane was incubated with peroxidase-conjugated goat anti-mouse Ig (Tago) diluted 1:100 in PBS-Tween™ for 2 hours at 22 °C. The blot was washed and developed with 4-chloro-1-naphtol as substrate.

Having generally described this invention, a further understanding can be obtained by reference to certain specific examples which are provided herein for purposes of illustration only and are not intended to be limiting unless otherwise specified.

### EXAMPLE 1

PREPARATION OF SOLUBLE 4-1BB IMMUNOGLOBULIN CHIMERA

For the identification and characterization of 4-1BB ligand(s) a soluble immunoglobulin chimera of 4-1BB was prepared. This recombinant globulin (Rg) was used in immunohistochemical studies to identify tissues expressing the 4-1BB ligand. The fusion gene encoding the 4-1BB Rg chimera was prepared by genetic fusion of a cDNA fragment encoding the extracellular domain of 4-1BB to a genomic DNA fragment encoding the hinge, CH2, and CH3 domains of a human IgGl and subcloned into a mammalian expression vector (Figure 1A). When transfected into COS cells this plasmid directs the expression and secretion of the 4-1BB Rg. The 4-1BB Rg is produced as a disulfide-linked homodimer (Figure 1B) and accumulates in the supernatant of transfected cells in concentrations of ∼ 14 mg/l. The 4-1BB Rg protein used in these studies was recovered from the supernatant by affinity chromatography on a protein A column. As a control for nonspecific and Fc-mediated binding a CD40 Rg chimera was used or human IgG1. The human B cell surface protein CD40 is distantly related to 4-1BB (Mallett, S. et al. (1991) Immunol. Today 12:220-223).

### EXAMPLE 2

TISSUE DISTRIBUTION OF THE 4-1BB LIGAND

Immunohistology studies with the 4-1BB Rg showed broad reactivity with a number of tissues including kidney, liver, epidermis and dermis of skin, in addition to primary and secondary lymphoid tissues. Isotype matched human IgG1 control showed no binding (Figure 2). Within the thymus, 4-1BB Rg bound to both lymphoid and stromal elements in the cortex and medulla, with more extensive labeling of the medulla. In the spleen, 4-1BB Rg bound red pulp more extensively than white pulp.

The widespread distribution of ligand(s) for the 4-1BB Rg in tissues of epithelial or mesenchymal derivation raised the possibility that extracellular matrix components may function as ligands.

### EXAMPLE 3

EXTRACELLULAR MATRIX PROTEINS ARE LIGANDS FOR 4-1BB

To investigate the possible interaction between 4-1BB and extracellular matrix proteins, the complete 4-1BB protein was expressed in a heterologous system and its ability to bind to extracellular matrix proteins was examined. A cDNA fragment encoding the complete 4-1BB protein was isolated from a murine T cell cDNA library, subcloned into the mammalian expression vector CDM8 (Seed, B. (1987) Nature 329:840-843) and transfected into COS cells. Biotin-conjugated fibronectin, laminin, vitronectin and collagen VI bound to COS cells expressing 4-1BB whole biotin-conjugated collagen I and BSA or the phycoerythrin-conjugated strept-avidin alone did not (Figure 2E-H). COS cells expressing CD40 (Stamenkovic, I. (1989) EMBO J 8:1403-1410) showed no binding to any of the purified extracellular matrix or control proteins.

To evaluate the relative affinity of the interaction and extracellular matrix proteins, 4-1BB Rg was immobilized on plastic coated with anti-human IgG antibodies and its ability to bind to increasing concentrations of extracellular matrix proteins was tested (Figure 3). The binding of fibronectin, laminin, vitronectin, and collagen VI to 4-1BB was saturable and half maximal binding was reached at concentrations of ∼ 5.0, 1.5, 1.0 and 2.0 µg/ml, respectively. Collagen I, BSA and alkaline phosphatase-conjugated avidin alone showed no binding to all of the extracellular matrix proteins and control proteins tested (Figure 3).

### EXAMPLE 4

4-1BB-EXTRACELLULAR MATRIX PROTEIN INTERACTIONS ARE NOT MEDIATED BY RGD OR CS-1 SEOUENCES

A number of proteins which mediate the interaction between lymphocytes and the extracellular matrix have been identified, including the integrins which have been shown to bind to fibronectin, laminin, vitronectin and collagen (Springer, T.A. (1990) Nature 346:425-434). In some instances, the interaction between integrins and extracellular matrix proteins has been shown to be mediated by Arg-Gly-Asp (RGD) sequences in the extracellular matrix proteins (Ruoslahti, E. et al. (1987) Science 238:491-497). For example, the interaction between fibronectin and the VLA-5 integrin has been shown to be RGD dependent (Rytela, R. et al (1985) Cell 40:191-198), while the interaction between fibronectin and the VLA-4 integrin has been shown to be mediated by an alternatively spliced domain of fibronectin (CS-1) (Wayner, E. et al. (1989) J. Cell Biol. 109:1321-1330; Guan, J.L. et al. (1990) Cell 60:53-61). To investigate the role of RGD and CS-1 sequences in the 4-1BB extracellular matrix protein binding, the ability of several RGD and CS-1 peptides to block the binding of fibronectin to 4-1BB Rg immobilized on plastic was tested. These peptides include RGD peptides which inhibit cell binding to either fibronectin or vitronectin, specifically, or RGD peptides which inhibit the binding to both fibronectin and vitronectin, or the CS-1 peptide, a scrambled version of the CS-1 peptide, or a mixture of RGD and CS-1 peptides. These peptides and peptide combinations had little or no effect on 4-1BB Rg-fibronectin adhesion even at concentrations 10X greater than those which inhibit the fibronectin-VLA-4 interaction (Nojim, Y. et al. (1990) J. Exp. Med. 172:1185-1192).

In an effort to identify the region or regions of the fibronectin molecule responsible for mediating the 4-1BB fibronectin interaction, a number of blocking studies were carried out with six overlapping proteolytic fragments of fibronectin (Figure 4A) (Ruoslahti, E. (1988) Ann. Rev. Biochem. 57:375-413). Three of these, T30, T60 and C120, blocked the binding of fibronectin to immobilized 4-1BB Rg in a concentration dependent fashion, the C45 peptide had a partial blocking effect and C40 and P50 had no blocking effect (Figure 4B). This experiment provides evidence that multiple sites within fibronectin mediate the 4-1BB-fibronectin interaction.

### EXAMPLE 5

THE EXTRACELLULAR MATRIX PROTEIN BINDING SITES ON 4-1BB ARE OVERLAPPING

To determine whether the interaction between the different extracellular matrix proteins and 4-1BB was mediated by the same regions of the 4-1BB protein, blocking experiments were carried out with the T30 and T60 fibronectin proteolytic fragments. The T30 and T60 fragments were found to block the binding of laminin, vitronectin, and collagen VI to immobilized 4-1BB Rg in a concentration dependent fashion (Figure 4C).

### EXAMPLE 6

4-1BB ADHESION IS INHIBITED BY GLYCOSAMINOGLYCANS

In light of the inability of RGD or CS-1 peptides to interfere with the interaction between 4-1BB Rg and extracellular matrix components, the basis for this interaction was studied further. Because proteoglycans have been shown to bind to extracellular matrix proteins and growth factors by a largely RGD-independent mechanism (Ruoslahti, E. et al. (1990) Cell 64:867-869; Ruoslahti, E. (1988) Ann. Rev. Cell Biol. 4:229-255), we examined the effects of glycosaminoglycans on 4-1BB interactions with extracellular matrix components and determined whether 4-1BB was proteoglycan. The ability of glycosaminoglycans as well as simple sugars to inhibit the interaction between extracellular matrix proteins and 4-1BB was examined using COS cell transfectants expressing 4-1BB. The glycosaminoglycans heparin, hyaluronan and chondroitin sulfate partially inhibited the binding of extracellular matrix proteins to 4-1BB, while de-sulfated heparin had no effect (Figure 5A). The interaction between extracellular matrix proteins and 4-1BB was also inhibited by the anionic carbohydrate polymers fucoidan and dextran sulfate (Figure 5A). The simple carbohydrates fucose, galactose, mannose, glucose, galactose-6-sulfate, N-acetyl-galactosamine, N-acetyl-glucosamine, and N-acetyl-neuraminic acid had little or no effect on the 4-1BB-fibronectin interaction.

4-1BB contains no Ser-Gly sequences which are putative for chondroitin/dermatan sulfate attachment (Doege, K. et al. (1987) J. Biol. Chem. 262:17757-17767; Oldberg, A. et al. (1987) Biochem. J. 243:255-259) and SDS-PAGE analysis of 4-1BB Rg treated with chondroitin ABC lyase, heparinase, heparatinase, and hyaluronidase showed no evidence for the presence of these glycosaminoglycans (Figure 5B). Digestion with keratinase (endo-β-galactosidase) reduced the molecular mass of 4-1BB Rg (Figure 5B) and 4-1BB Rg reacted with the anti-keratan sulfate monoclonal antibody 5D4 (Caterson, B. et al. (1983) J. Biol. Chem. 258:8848-8854) suggesting that 4-1BB Rg is modified with carbohydrate moieties containing the sequence GlcNac(β-1, 3)Gal(β-1,4)GlcNAc (Fukuda, M.N. (1981) J. Biol. Chem. 256:3900-₃905) and possibly keratan sulfate. Parallel digests with N-glycanase, O-glycanase and neuraminidase demonstrate that 4-1BB Rg contains N-linked and O-linked carbohydrates as well as sialic acid (Figure 5B).

The foregoing description and Examples are intended as illustrative of the present invention, but not as limiting. Numerous variations and modifications may be effected without departing from the true spirit and scope of the present invention.

## Claims

1. A method of modulating leukocyte function comprising inhibiting the interaction between leukocyte receptors and extracellular matrix associated proteins on tissues with a soluble lymphocyte ligand protein such that said lymphocyte ligand protein binds to said extracellular matrix associated proteins and blocks interaction with said leukocytes.

2. The method of Claim 1, wherein said lymphocyte ligand protein is a member of the nerve growth factor receptor superfamily.

3. The method of Claim 2, wherein said protein is a soluble 4-1BB protein.

4. The method of Claim 2, wherein said protein is a soluble CD27.

5. The method of Claim 2, wherein said protein is a soluble CD30.

6. The method of Claim 2, wherein said protein is a soluble CD40.

7. The method of Claim 2, wherein said protein is a soluble TNF.

8. A soluble lymphocyte receptor protein capable of inhibiting the interaction of leukocytes with extracellular matrix associated proteins.

9. A soluble lymphocyte ligand protein capable of inhibiting the interaction of leukocytes with extracellular matrix associated proteins.

10. The soluble protein of Claim 8 or Claim 9 comprising a 4-1BB fusion protein.

11. The soluble protein of Claim 10, wherein the 4-1BB fusion protein is 4-1BB-Rg.

12. The soluble protein of Claim 8 or Claim 9 comprising a CD27 fusion protein.

13. The soluble protein of Claim 12, wherein the CD27 fusion protein is CD27 Rg.

14. The soluble protein of Claim 8 or Claim 9 comprising a CD30 fusion protein.

15. The soluble protein of Claim 14, wherein the CD30 fusion protein is CD30 Rg.

16. The soluble protein of Claim 8 or Claim 9 comprising a FAS fusion protein.

17. The soluble protein of Claim 16, wherein the FAS fusion protein is FAS Rg.

18. Use of a soluble protein according to any one of Claims 8 top 17 in the preparation of a composition for use in the modulation of leukocyte function by inhibiting the interaction between leukocyte receptors and extracellular matrix proteins, wherein said soluble protein binds to the extracellular matrix protein and blocks interaction with the leukocytes.

19. Use according to Claim 18, wherein said soluble protein is a member of the nerve growth factor receptor superfamily.

20. A soluble protein according to any one of Claims 8 to 17 for use in therapy.


SEQUENCE LISTING

(1) GENERAL INFORMATION:

(i) APPLICANT:
(A) NAME: Bristol-Myers Squibb Company
(B) STREET: 3005 First Avenue
(C) CITY: Seattle
(D) STATE: Washington
(E) COUNTRY: USA
(F) POSTAL CODE: 98121

(ii) TITLE OF INVENTION: EXTRACELLULAR MATRIX RECEPTOR LIGANDS THAT
MODULATE LEUKOCYTE FUNCTION

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(2) INFORMATION FOR SEQ ID NO:1:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

GCGACGCGTA AGCTTGTCCT GTGCATGTGA CATTTC 36

(2) INFORMATION FOR SEQ ID NO:2:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

CACTCGAGCG GCCGCTCACA GCTCATAGCC TCCTCC 36

(2) INFORMATION FOR SEQ ID NO:3:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

CCGCGGGTAC CCGTGCAGAA CTCCTGTGAT AACTGT 36

(2) INFORMATION FOR SEQ ID NO:4:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

CCGCTACGTA GGATCCTGCA AGGAGTCCCC TCCTGG 36